# EUROPÄISCHE PATENTSCHRIFT

(11) **EP 1 321 105 B1**
(45) Veröffentlichungstag und Bekanntmachung des Hinweises auf die Patenterteilung: **20.08.2003**
(21) Anmeldenummer: 01129381.8
(22) Anmeldetag: 18.12.2001
(51) Int. Cl.: A61B 19/00

(54) **Projektion von Patientenbilddaten aus Durchleuchtungs bzw. Schichtbilderfassungsverfahren auf Oberflächenvideobilder**
Superposition of patient X-ray or scanning imaging data and superficial video images
Superposition de données d'imagerie de rayons X ou tomographique et d'images vidéo superficielles

(43) Veröffentlichungstag der Anmeldung: 25.06.2003
(60) Teilanmeldung: 03008762.1
(73) Patentinhaber: BrainLAB AG, 85551 Kirchheim/Heimstetten (DE)
(72) Erfinder: Vilsmeier, Stefan, 6330 Kufstein (AT)
(74) Vertreter: Schwabe - Sandmair - Marx

(56) Entgegenhaltungen:
- EP-A- 0 672 389
- WO-A-96/20421
- US-A- 5 694 142
- US-A- 5 765 561
- US-A- 6 038 467

## Beschreibung

Die vorliegende Erfindung betrifft eine Vorrichtung zur Projektion von Patientenbilddaten aus Durchleuchtungs- bzw. Schichtbilderfassungsverfahren auf Videobilder. Die Vorrichtung umfasst eine Bildanzeigevorrichtung, mindestens eine Videokamera und ein computergestütztes Navigationssystem, das die Raumposition der Bildanzeigevorrichtung und/oder der Videokamera sowie die Raumpositionen eines Patientenkörperteils über dort angebrachte Trackingeinrichtungen erfassen kann.

Solche Vorrichtungen dienen dazu, einen Mediziner bei der Diagnose bzw. bei der Behandlung eines Patienten visuell zu unterstützen. Es soll die Möglichkeit geschaffen werden, Bilder aus dem Inneren des Patienten mit herkömmlichen Videobildern zu verknüpfen, um Behandlung und Diagnose zu erleichtern.

Aus der US-A-5,765,561 ist ein videobasiertes chirurgisches Zielsystem bekannt, für das unter anderem die Verwendung einer getrackten Videokamera vorgeschlagen wird, deren Bilder auf dem stationär angeordneten Bildschirm eines Navigationssystem mit zugeordneten Bildern aus Durchleuchtungs- bzw. Schichtbilderfassungsverfahren überlagert werden. Nachteilig an einer solchen Ausführungsform ist insbesondere, dass der Arzt, der die Bilder betrachten möchte, immer wieder vom Patienten weg und zum Bildschirm des Navigationssystem hinblicken muss. Dabei kann er nicht mehr genau darauf achten, in welcher Stellung nun die Kamera steht, also von welcher Außenposition genau er die überlagerten Bilder erhält. Das System ist deshalb etwas umständlich und ermöglicht keinen direkten Einblick in den Patientenkörperteil.

Es ist die Aufgabe der vorliegenden Erfindung, eine Vorrichtung zur Projektion von Patientenbilddaten aus Durchleuchtungs- bzw. Schichtbilderfassungsverfahren auf Videobilder bereitzustellen, welche die oben genannten Nachteile des Standes der Technik überwindet. Insbesondere soll es dem Behandlungspersonal möglich sein, direkt und unmittelbar in Ansicht des Patienten die gewünschten Bilddaten zu erhalten.

Diese Aufgabe wird erfindungsgemäß dadurch gelöst, dass die Bildanzeigevorrichtung und die Videokamera einander zugeordnet und als tragbare Einheit ausgebildet sind. Mit dieser erfindungsgemäßen Ausgestaltung ergibt sich nun die Möglichkeit für den behandelnden Arzt, die Bildanzeigevorrichtung selbst mit zugeordneter Kamera vor das Patientenkörperteil zu halten und damit im unmittelbaren Aufblick auf den Patienten die gewünschten anatomischen Informationen auszulesen. Dabei ist es möglich, im auf der Bildanzeigevorrichtung dargestellten Bild das Videobild oder das Bild aus dem Durchleuchtungs- bzw. Schichtbilderfassungsverfahren je nach Wunsch mehr oder weniger transparent einzustellen, um so gerade die gewünschten Informationen am deutlichsten in der Gesamtanzeige hervorzuheben. Mit der erfindungsgemäßen Vorrichtung wird ein weiterer Schritt in die Richtung getan, einen "gläsernen Patienten" zu schaffen, und zwar einfach dadurch, dass man die Bildanzeigevorrichtung vor das in Frage kommende Patientenkörperteil hält. Da die Kamerazuordnung zur Bildanzeigevorrichtung über die Trackingeinrichtungen verfolgt wird, ist die Position dieser beiden Elemente immer bekannt, und das Navigationssystem hat Informationen darüber, wie und aus welcher Richtung der Patientenkörperteil angesehen wird. Insbesondere bei der Durchführung minimal invasiver Eingriffe, bei denen keine Freilegung des Eingriffsgebietes erfolgt, bietet die erfindungsgemäße Vorrichtung somit die Möglichkeit für den behandelnden Arzt, sich während der Operation nochmals genau über die Lage der Anatomieteile des Patienten in Kenntnis zu setzen. Auf der Bildanzeigevorrichtung können dabei natürlich auch getrackte bzw. navigierte Instrumente angezeigt werden. Der Arzt muss seinen Blick nicht mehr vom Patienten abwenden, um in erfindungsgemäßer Weise in das Innere des Patientenkörpers hineinzusehen.

Gemäß einer bevorzugten Ausführungsform der Erfindung ist die Videokamera auf der Rückseite der Bildanzeigevorrichtung angeordnet. Dabei entsteht ein kompaktes Gerät; die Kamera kann vollständig aus dem Blickbereich des Benutzers herausgenommen werden. Grundsätzlich ist anzumerken, dass die Kamera an jeder Stelle der Rückseite der Bildanzeigevorrichtung angebracht, oder in dieser integriert sein kann, zum Beispiel auch im Randbereich der Bildanzeigevorrichtung.

Bevorzugt steht die Videokamera in vorbestimmter bzw. systembekannter räumlicher Zuordnung zur Bildanzeigevorrichtung und die Trackingeinrichtung ist an der Bildanzeigevorrichtung angeordnet. Durch die vorbestimmte bzw. systembekannte räumliche Zuordnung von Videokamera und Bildanzeigevorrichtung wird die Möglichkeit eröffnet, nur eines dieser beiden Geräte mittels der Trackingeinrichtung zu verfolgen, da man immer genau weiß, wo sich dann das andere Gerät befindet. Es steht dem Konstrukteur dann auch frei, die Trackingeinrichtung entweder an der Bildanzeigevorrichtung selbst oder an der Videokamera anzuordnen, je nachdem wie diese Trackingeinrichtung am bestem vom Navigationssystem erfasst werden kann. Bei optisch basierten Navigationssystemen kann die Trackingeinrichtung eine reflektierende oder aktiv abstrahlende Markeranordnung sein, während es natürlich im Rahmen der vorliegenden Erfindung auch möglich ist, die Navigation über ein Magnettrackingsystem durchzuführen, bei dem Spulen in einem erzeugten Magnetfeld verfolgt werden.

Um die Bildanzeigevorrichtung leicht und gut handhabbar auszugestalten, kann sie als tragbarer LCD-Flachbildschirm ausgestaltet werden.

Die Datenübertragung zwischen den einzelnen Geräten der erfindungsgemäßen Vorrichtung kann in unterschiedlicher Weise realisiert werden. Einerseits ist es möglich, die auf die Videobilder zu projizierenden Bilddaten aus dem Durchleuchtungs- bzw. Schichtbilderfassungsverfahren über Funkschnittstellen vom Navigationssystem zur Bildanzeigevorrichtung zu übermitteln, während andererseits auch eine Übermittlung über Datenkabel denkbar ist. Insbesondere vorteilhaft sollte sich im Rahmen der Handhabung der erfindungsgemäßen Vorrichtung eine Ausgestaltung erweisen, bei der die Bildanzeigevorrichtung mit eigener Energieversorgung (Batterie bzw. Akku) versehen ist und eine Funkübertragung erfolgt, da damit die weitgehendste Handhabungsfreiheit erreicht wird.

Die Videokamera sollte gemäß einer bevorzugten Ausführungsform eine kleine Blende und eine geringe Schärfentiefe aufweisen, damit nur ein kleiner Bereich des erfassten Bildes in der Fokalebene liegt. Mit einer solchen Kamera ist es möglich, den Abstand zwischen dem Bild und der Fokalebene zu ermitteln. Wenn die Raumposition der Kamera aus dem Navigationssystem her bekannt ist und der Abstand zur Bildebene ebenfalls bekannt ist, kann das dem Navigationssystem angeschlossene Computersystem die räumliche Position des Videobildes in Echtzeit errechnen. Damit wird eine Zuordnung von Videobild und Bilddaten aus einer vorher durchgeführten Durchleuchtungs- bzw. Schichtbilderfassung optimal möglich.

Gemäß einer weiteren vorteilhaften Ausgestaltung der erfindungsgemäßen Vorrichtung ist an der Videokamera bzw. an der Bildanzeigevorrichtung eine Datenübertragungseinrichtung angeordnet, mittels der die Informationen über das auf der Bildanzeigevorrichtung angezeigte Bild an das Navigationssystem übertragen werden. Die vom Navigationssystem an die Bildanzeigevorrichtung übermittelten, zu projizierenden Bilddaten werden dann in Größe und Position auf der Bildanzeigevorrichtung so angepasst, dass sie sich mit den Videobildern decken, also eine 1:1-Darstellung beider Bilder in Überdeckung entsteht. Im Navigationssystem kann dazu eine Konturen-Matching-Einheit bereitgestellt werden, mittels der die Überdeckung der Videobilder und der projizierten Bilder realisiert wird, insbesondere über Außenkonturen-Matching für den Patientenkörperteil.

Es soll hier noch angemerkt sein, dass es im Rahmen der Erfindung denkbar und vorteilhaft sein kann, an der tragbaren Einheit verschiedenste Steuerungs- bzw. Befehleingabegeräte für die Veränderung des angezeigten, kombinierten Bildes anzubringen. Beispielsweise kann die Fokalebene sowohl der eingespielten Durchleuchtungs- oder Schichtbilddaten als auch des angezeigten Videobildes durch solche Steuerungselemente zu verändern sein. Wie oben schon angesprochen ist es auch möglich, Steuerungselemente vorzusehen, die den jeweiligen Transparenzgrad eines der Bilder gegenüber dem anderen ändern.

An der Kamera bzw. in deren Umgebung an der Bildanzeigevorrichtung ist vorteilhafterweise eine Beleuchtungsvorrichtung für den Patientenkörperteil vorgesehen, speziell beispielsweise als Ringlicht (LEDs, Lampen, Leuchtstoffröhren).

Die Erfindung wird im Weiteren anhand einer Ausführungsform näher erläutert. In den hierzu bereitgestellten Zeichnungen zeigen:
- Figur 1: eine schematische Darstellung für die Verwendung einer erfindungsgemäßen Vorrichtung zur Ansicht eines Patienten-Kniegelenks in der Aufsicht;
- Figur 2: eine seitliche Ansicht der Vorrichtung nach Figur 1 mit schematisch dargestelltem Navigationssystem; und
- Figur 3: ein Ablaufdiagramm für den Betrieb der erfindungsgemäßen Vorrichtung.

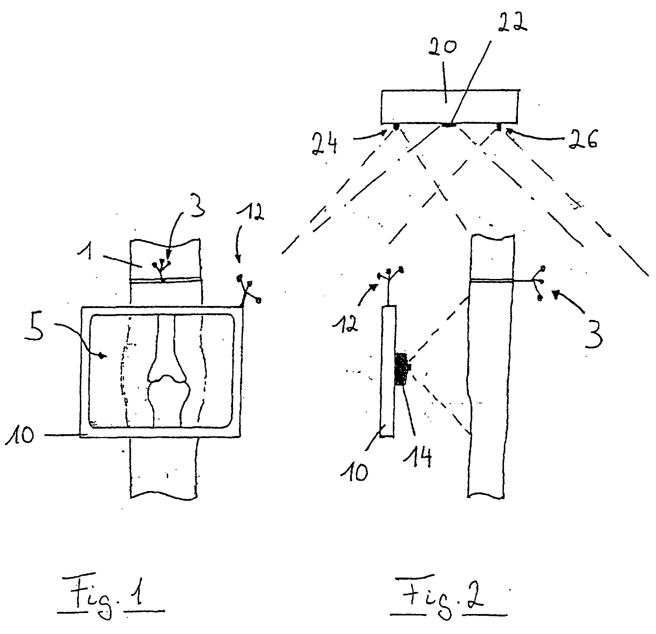

In der Figur 1 ist schematisch eine erfindungsgemäße Vorrichtung dargestellt, mit der beispielsweise in das Innere eines Gelenks eines Patienten Einsicht genommen werden kann. Das Bein 1 des Patienten soll am Gelenk 5 untersucht werden. Hierzu wird der erfindungsgemäß ausgestaltete Flachbildschirm 10 direkt vor das Gelenk gehalten, und auf dem Schirm 10 erscheint dann ein Bild, das sowohl Bildinformationen aus dem Videokamerabild der Kamera 14 (Figur 2) enthält als auch Bildinformationen über die innere anatomische Struktur, welche beispielsweise per Funkübertragung von einem Navigationsgerät übermittelt werden. Dazu ist vorab von dem Patienten bzw. von dessen Bein 1 eine Schichtbildaufnahme (MR- oder CT-Aufnahme) erstellt worden.

Wie besser in Figur 2 deutlich wird, befindet sich die Kamera 14 fest angebracht an der Rückseite des Bildschirms 10.

Möglich wird diese Art der Bilddarstellung dadurch, dass die Position der Kamera 14 bzw. des Bildschirms 10 im Raum um das Bein 1 des Patienten herum mittels einer Trackingeinrichtung ermittelt wird. Diese Trackingeinrichtung ist im vorliegenden Fall eine optische Trackingeinrichtung, nämlich eine Markeranordnung 12, deren Position mittels des schematisch dargestellten Navigationssystems 20 erfasst wird. Das Navigationssystem 20 umfasst hierzu beispielsweise zwei beabstandete Infrarot-Kameras 24, 26 und einen Infrarot-Lichtabstrahler 22. Aus den beiden Bildern der Infrarot-Kamera 24, 26 kann die dreidimensionale Raumposition der Markeranordnung 12 und damit auch des Bildschirms 10 und der fest installierten Kamera 14 ermittelt werden. Es ist ferner möglich die Raumposition des Beines 1 durch eine entsprechende Markeranordnung 3 im Raum zu ermitteln.

Wenn nun die Position von Bildschirm 10 und Kamera 14 bekannt ist, kann in folgender Weise die Position der aktuellen Bildebene erfasst werden. Die Kamera 14 hat nämlich eine kleine Blende und eine geringe Schärfentiefe, so dass nur das Bild in einer bestimmten Fokalebene scharf erscheint. Es lässt sich hierdurch die Position dieser Bildebene ermitteln und auf dem Bildschirm 10 ein Bild aus dem CT- oder MR-Aufnahmeverfahren übertragen, welche ebenfalls gerade in dieser Bildebene liegt. Wichtig ist hierbei, dass das Abbildungsverhältnis des Videobildes in einem 1:1-Maßstab zum Schichtaufnahmebild angezeigt wird. Wenn nun mit der erfindungsgemäßen Vorrichtung das Gelenk eines Patienten betrachtet wird, sieht man beispielsweise am oberen und unteren Bildschirmrand die Konturen des Patientenbeines. Mit dieser Information kann nunmehr das Bild aus dem Schichtaufnahmeverfahren so angepasst werden, dass es gerade über dem Videobild liegt, um einen realistischen Eindruck zu gewährleisten. Der Betrachter des Bildschirms 10 kann dann auf dem Bildschirm sowohl das Videobild, also ein echtes Bild, ansehen, als auch das "innere" Bild aus dem Schichtaufnahmeverfahren, und zwar in jeweils gewünschter Tiefe und in jeweils eingestellter Transparenz für beide Bilder. Diese Einstellungen können über nicht dargestellte Steuerungsorgane, zum Beispiel Kippschalter an der Bildschirmvorderseite, getroffen werden.

Die erfindungsgemäße Vorrichtung gibt damit dem Betrachter erstmals die Möglichkeit, in direktem Aufblick auf den Patienten in Echtzeit in den inneren Behandlungsbereich hineinzusehen und die Behandlung entsprechend zu planen oder anzupassen. Er muss seinen Blick nicht mehr vom Patienten abwenden und auf einen fest installierten Navigationsbildschirm sehen, was insbesondere von Vorteil ist, wenn die Reaktionen des Patienten auf bestimmte ärztliche Maßnahmen überprüft werden sollen. Da die "inneren" Bilder aus den Durchleuchtungs- bzw. Schichtbildaufnahmeverfahren so gestaltet werden können, dass sie virtuell 3-D-Ansichten der inneren Körperteile darstellen, kann das Bild insgesamt sehr anschaulich gemacht werden.

Obwohl also nicht vor Ort ein Durchleuchtungs- bzw. Schichtbild aufgenommen wird, entsteht somit ein virtuell "gläserner Patient".

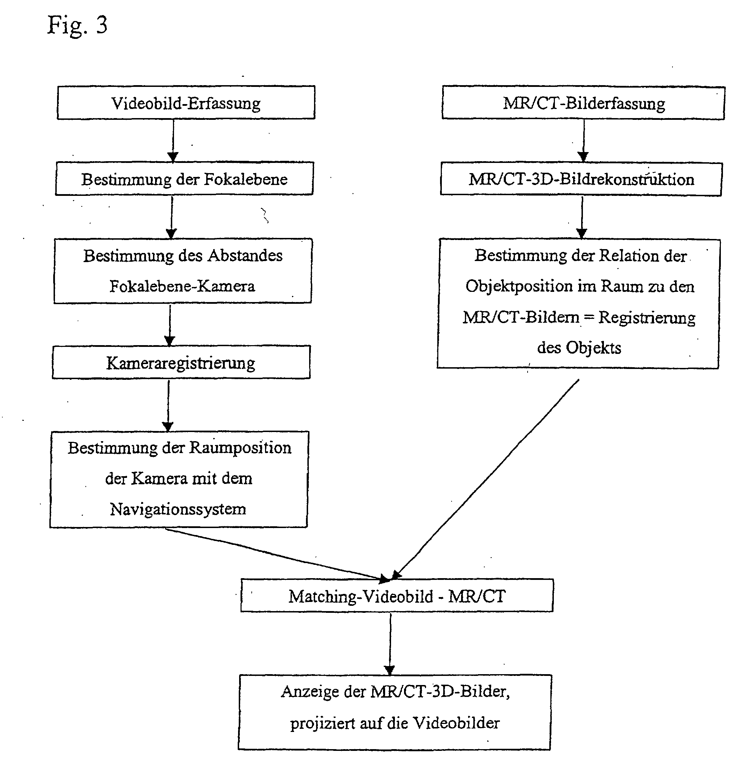

In der Figur 3 ist der Ablauf eines Verfahrens aufgezeigt, wie es mit der erfindungsgemäßen Vorrichtung durchgeführt wird, insbesondere wird hierbei die vom Computer des Navigationssystems durchgeführte Tätigkeit beleuchtet. Die obere linke Seite des Ablaufdiagramms betrifft die Vorgänge bei der Videobilderfassung, während auf der rechten Seite die Bilderfassung für das Körperinnere erläutert wird, und zwar am Beispiel einer MR-/CT-Bilderfassung. Bei der Videobilderfassung wird zunächst die Fokalebene bestimmt, wie dies vorher schon erläutert wurde. Danach erfolgt eine Bestimmung des Abstandes der Fokalebene zur Kamera, worauf die Kamera mittels ihrer Trackingeinrichtung im Navigationssystem registriert wird. Es ist nun die Bestimmung der Raumposition der Kamera gegenüber dem Navigationssystem bekannt.

Was die MR-/CT-Bilderfassung betrifft, so wird diese zunächst vorab erfolgen, worauf aus den erhaltenen Daten eine dreidimensionale Bildrekonstruktion erfolgt. Dann wird die Registrierung des Objektes, beispielsweise eines Patientenkörperteils, im Raum durchgeführt, d. h. es erfolgt eine Bestimmung der Relation der Objektposition im Raum zu den MR-/CT-Bildern. Realisiert werden kann dies durch die räumliche Erfassung einer am Objekt befindlichen Markierungsanordnung.

Wenn die oben aufgeführten Schritte der Videobilderfassung und MR-/CT-Bildverarbeitung durchgeführt worden sind, erfolgt nunmehr ein Videobild-/MR-/CT-Bild-Matching, d. h. die beiden Bilder werden computergesteuert in Übereinstimmung gebracht, was die Bildposition und die Bildgröße betrifft. Danach können die MR-/CT-3D-Bilder projiziert auf die Videobilder auf der Bildanzeigevorrichtung überlagert werden und den behandelnden Arzt bei der Diagnose bzw. Behandlung unterstützen.

## Patentansprüche

1. Vorrichtung zur Projektion von Patientenbilddaten aus Durchleuchtungs- bzw. Schichtbilderfassungsverfahren auf Videobilder, mit einer Bildanzeigevorrichtung (10), mindestens einer Videokamera (14) und einem computergestützten Navigationssystem, das die Raumpositionen der Bildanzeigevorrichtung (10) und/oder der Videokamera (14) sowie die Raumpositionen eines Patientenkörperteils (1) über dort angebrachte Trackingeinrichtungen (12, 3) erfassen kann, **dadurch gekennzeichnet, dass** die Bildanzeigevorrichtung (10) und die Videokamera (14) einander zugeordnet und als tragbare Einheit ausgebildet sind.

2. Vorrichtung nach Anspruch 1, **dadurch gekennzeichnet, dass** die Videokamera (14) auf der Rückseite der Bildanzeigevorrichtung (10) angeordnet ist.

3. Vorrichtung nach einem der Ansprüche 1 oder 2, **dadurch gekennzeichnet, dass** die Videokamera (14) in vorbestimmter bzw. systembekannter räumlicher Zuordnung zur Bildanzeigevorrichtung (10) steht und die Trackingeinrichtung (12) an der Bildanzeigevorrichtung (10) angeordnet ist.

4. Vorrichtung nach einem der Ansprüche 1 oder 2, **dadurch gekennzeichnet, dass** die Videokamera (14) in vorbestimmter bzw. systembekannter räumlicher Zuordnung zur Bildanzeigevorrichtung (10) steht und die Trackingeinrichtung an der Videokamera (14) angeordnet ist.

5. Vorrichtung nach einem der Ansprüche 1 bis 4, **dadurch gekennzeichnet, dass** die Bildanzeigevorrichtung ein tragbarer LCD-Flachbildschirm ist.

6. Vorrichtung nach einem der Ansprüche 1 bis 5, **dadurch gekennzeichnet, dass** die auf die Videobilder zu projizierenden Bilddaten aus den Durchleuchtungs- bzw. Schichtbilderfassungsverfahren über Funkschnittstellen vom Navigationssystem zur Bildanzeigevorrichtung übermittelt werden.

7. Vorrichtung nach einem der Ansprüche 1 bis 5, **dadurch gekennzeichnet, dass** die auf die Videobilder zu projizierenden Bilddaten aus den Durchleuchtungs- bzw. Schichtbilderfassungsverfahren über ein Datenkabel vom Navigationssystem zur Bildanzeigevorrichtung übermittelt werden.

8. Vorrichtung nach einem der Ansprüche 1 bis 7, **dadurch gekennzeichnet, dass** die Videokamera (14) eine kleine Blende und eine geringe Schärfentiefe aufweist.

9. Vorrichtung nach einem der Ansprüche 1 bis 8, **dadurch gekennzeichnet, dass** an der Videokamera (14) bzw. an der Bildanzeigevorrichtung (10) eine Datenübertragungseinrichtung angeordnet ist, mittels der Informationen über das auf der Bildanzeigevorrichtung angezeigte Bild an das Navigationssystem übertragen werden, wobei die vom Navigationssystem an die Bildanzeigevorrichtung übermittelten, zu projizierenden Bilddaten aus den Durchleuchtungs- bzw. Schichtbilderfassungsverfahren in Größe und Position auf der Bildanzeigevorrichtung so angepasst werden, dass sie sich mit den Videobildern decken.

10. Vorrichtung nach Anspruch 9, **dadurch gekennzeichnet, dass** das Navigationssystem eine Konturen-Matching-Einheit aufweist, mittels der die Überdeckung der Videobilder und der projizierten Bilder realisiert wird, insbesondere über Außenkonturen-Matching für den Patientenkörperteil (1).

11. Vorrichtung nach einem der Ansprüche 1 bis 10, **dadurch gekennzeichnet, dass** an der Kamera (14) bzw. in deren Umgebung an der Bildanzeigevorrichtung (10) eine Beleuchtungsvorrichtung für den Patientenkörperteil (1) vorgesehen ist; insbesondere ein Ringlicht aus LEDs, Lampen oder Leuchtstoffröhren.

## Claims

1. A device for projecting patient image data from radioscopic imaging methods and/or tomographic imaging methods onto video images, comprising an image display device (10), at least one video camera (14) and a computer-assisted navigation system which can detect the spatial positions of said image display device (10) and/or said video camera (14) and the spatial positions of a part of a patient's body (1) via tracking means (12, 3) attached to it, **characterised in that** said image display device (10) and said video camera (14) are assigned to each other and are realised as a portable unit.

2. The device as set forth in claim 1, **characterised in that** said video camera (14) is arranged on the rear side of said image display device (10).

3. The device as set forth in any one of claims 1 or 2, **characterised in that** said video camera (14) is spatially assigned to said image display device (10) in a way which is predetermined and/or known to the system, and **in that** said tracking means (12) is arranged on said image display device (10).

4. The device as set forth in any one of claims 1 or 2, **characterised in that** said video camera (14) is spatially assigned to said image display device (10) in a way which is predetermined and/or known to the system, and **in that** said tracking means (12) is arranged on said video camera (14).

5. The device as set forth in any one of claims 1 to 4, **characterised in that** said image display device (10) is a portable LCD flat screen.

6. The device as set forth in any one of claims 1 to 5, **characterised in that** the image data from said radioscopic imaging method and/or tomographic imaging method to be projected onto said video images are communicated to said image display device (10) via radio interfaces of said navigation system.

7. The device as set forth in any one of claims 1 to 5, **characterised in that** the image data from said radioscopic imaging method and/or tomographic imaging method to be projected onto said video images are communicated to said image display device (10) via a data cable of said navigation system.

8. The device as set forth in any one of claims 1 to 7, **characterised in that** said video camera (14) exhibits a small aperture and a low depth of field.

9. The device as set forth in any one of claims 1 to 8, **characterised in that** a data transmission means is arranged on said video camera (14) or on said image display device (10), by means of which information on the image shown on said image display device (10) is transmitted to said navigation system, wherein the image data to be projected, from said radioscopic imaging methods and/or tomographic imaging methods, communicated from said navigation system to said image display device (10), are adapted in size and position on said image display device (10) such that they are in registration with said video images.

10. The device as set forth in claim 9, **characterised in that** said navigation system comprises a contour-matching unit which superimposes said video images and said projected images, in particular via outer contour matching of said part of the patient's body.

11. The device as set forth in any one of claims 1 to 10, **characterised in that** an illuminating device for said part (1) of the patient's body is provided on said camera (14) or in its vicinity on said image display device (10); in particular, a ring light consisting of LEDs, lamps or fluorescent tubes.

## Revendications

1. Dispositif de superposition de données d'imagerie de rayons X ou tomographique et d'images vidéo comprenant un dispositif d'affichage d'images, au moins une caméra vidéo (14) et un système de navigation assisté par ordinateur, qui peut détecter les positions dans l'espace du dispositif d'affichage d'images (10) et/ou de la caméra vidéo (14) ainsi que les positions dans l'espace d'une partie du corps d'un patient (1) via des dispositifs de suivi (12, 3) qui y sont installés, **caractérisé en ce que** le dispositif d'affichage d'images (10) et la caméra vidéo (14) sont mis en correspondance et forment une unité portative.

2. Dispositif suivant la revendication 1, **caractérisé en ce que** la caméra vidéo (14) est disposée à la face arrière du dispositif d'affichage d'images (10).

3. Dispositif suivant l'une des revendications 1 ou 2, **caractérisé en ce que** la caméra vidéo (14) est disposée dans une position spatiale relative prédéterminée ou connue du système par rapport au dispositif d'affichage d'images (10) et le dispositif de suivi (12) est disposé sur le dispositif d'affichage d'images (10).

4. Dispositif suivant l'une des revendications 1 ou 2, **caractérisé en ce que** la caméra vidéo (14) est disposée dans une position spatiale relative prédéterminée ou connue du système par rapport au dispositif d'affichage d'images (10) et le dispositif de suivi (14) est disposé sur la caméra vidéo (14).

5. Dispositif suivant l'une des revendications 1 à 4, **caractérisé en ce que** le dispositif d'affichage d'images est un écran plat LCD portatif.

6. Dispositif suivant Tune des revendications 1 à 5, **caractérisé en ce que** les données d'imagerie de rayons X ou tomographique à superposer aux images vidéo sont transmises du système de navigation au dispositif d'affichage d'images via des interfaces radio.

7. Dispositif suivant l'une des revendications 1 à 5, **caractérisé en ce que** les données d'imagerie de rayons X ou tomographique à superposer aux images vidéo sont transmises du système de navigation au dispositif d'affichage d'images via un câble de données.

8. Dispositif suivant l'une des revendications 1 à 7, **caractérisé en ce que** la caméra vidéo (14) présente un petit diaphragme et une faible profondeur de champ.

9. Dispositif suivant l'une des revendications 1 à 8, **caractérisé en ce qu'**un dispositif de transmission de données est fixé à la caméra vidéo (14) ou au dispositif d'affichage d'images (10), avec lequel des informations sur l'image affichée par le dispositif d'affichage d'images sont transmises au système de navigation, les données d'imagerie de rayons X ou tomographique à superposes transmises par le système de navigation au dispositif d'affichage d'images étant adaptées en taille et position au dispositif d'affichage d'images de telle façon qu'elles recouvrent les images vidéo.

10. Dispositif suivant la revendication 9, **caractérisé en ce que** le système de navigation présente une unité de mise en correspondance de contours, avec laquelle le recouvrement des images vidéo et des images superposées est réalisé, en particulier via mise en correspondance de contours pour la partie du corps du patient (1).

11. Dispositif suivant l'une des revendications 1 à 10, **caractérisé en ce qu'**un dispositif d'éclairage pour la partie du corps du patient (1) est prévu sur la caméra (14) ou à proximité de celle-ci sur le dispositif d'affichage d'images (10), en particulier un luminaire annulaire formé de LED, lampes ou tubes fluorescents.
